# EUROPEAN PATENT SPECIFICATION

(11) **EP 4 112 042 B1**
(45) Date of publication and mention of the grant of the patent: **18.02.2026**
(21) Application number: 21382581.3
(22) Date of filing: 30.06.2021
(51) Int. Cl.: A61K 9/00, A61K 9/107, A61K 31/196, A61K 47/34, A61P 19/02, A61P 21/00, A61P 29/00

(54) **NANOEMULSION COMPRISING DICLOFENAC**
NANOEMULSION MIT DICLOFENAC
NANOÉMULSION COMPRENANT DU DICLOFÉNAC

(43) Date of publication of application: 04.01.2023
(73) Proprietor: Haleon CH SARL, 1197 Prangins (CH)
(72) Inventor: Alonso, Maria José, 15782 Santiago de Compostela (ES); Teijeiro, Desirée, 15782 Santiago de Compostela (ES); Catanzano, Ovidio, 15782 Santiago de Compostela (ES); Berrecoso Cuña, Germán, 15782 Santiago de Compostela (ES)
(74) Representative: Haleon Patent Department

(56) References cited:
- WO-A1-2006/096955
- CA-A1- 3 091 186
- US-A1- 2011 275 717
- US-A1- 2012 093 882
- US-A1- 2016 038 414
- US-B1- 8 852 628
- SHAKEEL FAIYAZ ET AL: "Removal of diclofenac sodium from aqueous solution using water/Transcutol/ethylene glycol/Capryol-90 green nanoemulsions", JOURNAL OF MOLECULAR LIQUIDS, ELSEVIER, AMSTERDAM, NL, vol. 199, 30 August 2014 (2014-08-30), pages 102 - 107, XP029090940, ISSN: 0167-7322, DOI: 10.1016/J.MOLLIQ.2014.08.030
- PRASETYO BAYU EKO ET AL: "Formulation and Physical Evaluation of Castor Oil based Nanoemulsion for Diclofenac Sodium Delivery System", RESEARCH JOURNAL OF PHARMACY AND TECHNOLOGY, vol. 11, no. 9, 1 January 2018 (2018-01-01), India, pages 3861, XP055873038, ISSN: 0974-3618, Retrieved from the Internet <URL:http://dx.doi.org/10.5958/0974-360X.2018.00707.2> DOI: 10.5958/0974-360X.2018.00707.2
- CHAUHAN SHIVALI ET AL: "DEVELOPMENT AND IN VITRO CHARACTERIZATION OF NANOEMULSION EMBEDDED THERMOSENSITIVE IN-SITU OCULAR GEL OF DICLOFENAC SODIUM FOR SUSTAINED DELIVERY", INTERNATIONAL JOURNAL OF PHARMACEUTICAL SCIENCES AND RESEARCH, vol. 9, no. 6, 1 June 2018 (2018-06-01), IN, pages 2301, XP055873043, ISSN: 0975-8232, Retrieved from the Internet <URL:https://ijpsr.com/wp-content/uploads/2018/08/19-Vol.-9-Issue-6-June-2018-IJPSR-RA-9119.pdf> DOI: 10.13040/IJPSR.0975-8232.9(6).2301-14
- DAS DEBASHREE ET AL: "Formulation and Assessment of In Vivo Anti-Inflammatory Potential of Omega-3-Fatty Acid Loaded Self Emulsifying Nanoemulsion", CURRENT NANOMEDICINE, vol. 7, no. 1, 20 February 2017 (2017-02-20), pages 47 - 58, XP055873044, ISSN: 2468-1873, DOI: 10.2174/246818730666616092612

## Description

### FIELD OF THE INVENTION

This disclosure relates to nano-formulations for the topical delivery of diclofenac. Specifically, it relates to a novel nanoemulsion formulation comprising a combination of diclofenac (free acid) and diclofenac alkali metal salt.

### BACKGROUND TO THE INVENTION

Topical, transdermal products comprising diclofenac, a non-steroidal anti-inflammatory drug (NSAID) of the acetic acid class, are currently available to patients and consumers in Europe and other countries, and are widely used for their analgesic and anti-inflammatory properties, providing an advantage over oral NSAID formulations, inter alia due to reduced systemic adverse effects. However, it is very difficult to develop topical formulations that are pharmaceutically acceptable and at the same time exhibit consumer preferred attributes. Additionally, diclofenac is a particularly challenging ingredient to work with, and poses many challenges to the formulator. Improved topical compositions comprising diclofenac are needed that meet pharmaceutical, technological and consumer expectations.

### SUMMARY OF THE INVENTION

There is provided a nanoemulsion for topical drug delivery, comprising:
**a)** diclofenac alkali metal salt, in a concentration of about 0.7% to about 1.3% (w/w),
**b)** diclofenac free acid, in a concentration of about 0.7% to about 1.3% (w/w),
**c)** diethyleneglycol monoethyl ether, in a concentration of about 3% to about 7% (w/w),
**d)** a mixture of polyethylene glycol mono- and diesters of 12-hydroxystearic acid comprising about 20% to about 40% (w/w) free polyethylene glycol,
**e)** caprylic capric triglyceride with a caprylic acid percentage of about 55% to about 60% (w/w) and a capric acid percentage of about 40% to about 45% (w/w), and
**f)** water.

In one embodiment, the ratio of diclofenac alkali metal salt to diclofenac free acid is about 0.8 : 1.2 to about 1.2 : 0.8.

In one embodiment, the total concentration of diclofenac is from about 1.5% to about 2.5% (w/w).

In one embodiment, the nanoemulsion further comprises: **g)** a cationic surfactant, in a concentration of about 0.1% to about 0.3% (w/w).

In one embodiment, the nanoemulsion is self-emulsifying.

In one embodiment, the nanoemulsion is transparent.

In one embodiment, the nanoemulsion is alcohol-free.

In one embodiment, component g) is a quaternary ammonium compound.

In one embodiment, component g) is a C₈-C₁₆-alkyl trimethylammonium salt.

In one embodiment, component d) is a mixture of polyethylene glycol mono- and diesters of 12-hydroxystearic acid.

In one embodiment, component e) is Labrafac^{™} Lipophile WL1349.

In one embodiment, component d) is present in a concentration of about 20% to about 40% (w/w) and component e) is present in a concentration of about 2.5% (w/w) to about 7.25% (w/w).

In one embodiment, the nanoemulsion is for use in the treatment of joint pain, osteoarthritis, muscle pain, back pain and/or inflammation.

In one embodiment, the nanoemulsion is provided in a container comprising an applicator for direct application onto the skin.

### DETAILED DESCRIPTION OF THE INVENTION

To facilitate extended therapeutic efficacy, an important objective of the inventors has been to provide a composition which facilitates improved diclofenac penetration through the skin, into regions of interest for anti-inflammatory and anti-nociception effects. The skin, however, is a natural barrier, providing hydrophobic qualities to minimise percutaneous absorption of ionised drugs, such as diclofenac.

One way to improve penetration of diclofenac is through incorporation of higher concentrations of the active pharmaceutical ingredient (API). This however entails numerous difficulties. Higher amounts of API can lead to chemical instability due to a higher or lower pH. Higher amounts of API can also cause instability due to interaction with other ingredients of the formulation. The approach of raising API concentration is especially limited for diclofenac, due to the very limited solubility and high cristallisation tendency of diclofenac. Furthermore, higher API concentrations render products more expensive, and enlarge their environmental footprint. Inter alia for environmental reasons, it is desirable to achieve the highest possible penetration with the least concentration of active. Thereby, API usage could be reduced without compromising efficacy. Furthermore, exposure of the environment with "unused" active could be reduced.

Another way to achieve higher penetration of diclofenac is through incorporation of (higher) concentrations of permeation enhancer(s) in a formulation. However, permeation enhancer(s) can also cause skin irritation, and can, in some concentrations, even have a penetration retarding effect. Penetration enhancers can also lead to instability of multiphase topical systems. Additionally, product characteristics like rheology or appearance can be negatively impacted by inclusion of permeation enhancers. It would be preferred to provide a product that achieves high penetration of diclofenac even without the need to incorporate a permeation enhancer. But ideally, penetrations could nevertheless be successfully incorporated into the formulation to further enhance penetration of the API, without a negative impact on stability, product attributes and safety. Thereby, a product range with different strengths or potencies could be created, based on the base formulation.

Several challenges lie in the manufacture of topical products. Many methods of manufacture are cumbersome, expensive and waste(water) intensive, e.g. because they require numerous vessels. Another problem specific to multiphase systems is the energy impact of usual emulsification methods. Using (high) shear homogenization, especially for prolonged periods of time, is energy intensive. This leads to higher production cost and can also negatively impact stability because of higher temperatures and oxygen introduction. Therefore, there is a need for a product that can be manufactured in an economic and environmentally friendly method of manufacture, without high energy impact.

An additional challenge is to provide a stable composition suitable for commercial distribution and sale. The minimum stability profile requires overcoming challenges including the resistance to phase separation in multiphase systems, creaming, precipitation and flocculation.

Another challenge is to provide a topical product with pleasant attributes. Currently, consumers prefer non greasy, light products. Also preferred are clear, transparent or translucent topical products. Ideally the products should have pleasing spread characteristics, and dry quickly without leaving residue on the skin. The texture of the product needs to be pleasant to consumers. Cooling properties are often preferred, however incorporation of excipients with a cooling effect is also limited due to various formulation challenges.

CA 3 091 186 A1, US 2016/038414 A1 and US 2012/093882 A1 propose nanoemulsions for topical delivery comprising diclofenac sodium.

The JOURNAL OF MOLECULAR LIQUIDS, 2014, 199, 102-107, discloses a nanoemulsion comprising diclofenac sodium adsorbed onto the surface of water.

The RESEARCH JOURNAL OF PHARMACY AND TECHNOLOGY, 2018, vol. 11, no. 9, 3861,also discloses a nanomulsion of diclofenac sodium.

The INTERNATIONAL JOURNAL OF PHARMACEUTICAL SCIENCES AND RESEARCH, 2018, vol. 9, no. 6, 2301, discloses a nanomulsion embedded thermosensitive in-situ ocular gel comprising diclofenac sodium.

CURRENT NANOMEDICINE, 2017, vol. 7, no. 1, 47-58, discloses a self-emulsyfying nanoemulsion comprising diclofenac sodium.

WO 2006/096955 A1 discloses a diclofenac sodium cream in a form of emulsion, and US 8 852 628 B1 discloses a transdermal patch comprising disclofenac acid.

US 2011/275717 A1 discloses a topical formulation in a form of solution inter alia comprising diclofenac sodium and polyethylene glycol 12-hydroxystearate.

None of these prior art documents discloses a topical, transdermal diclofenac formulation addressing the challenges described above.

Consequently, there remains a need for a topical, transdermal diclofenac formulation that addresses one or more of the described challenges.

Unless indicated otherwise, concentrations are given in %(w/w).

The term "about" in relation to a numerical value x means, for example, x±10%, x±5%, x±4%, x±3%, x±2%, x±1%.

### PHARMACEUTICAL COMPOSITIONS

This disclosure provides pharmaceutical compositions for topical drug delivery which are nanoemulsions with an aqueous outer phase. Nanoemulsions are disperse systems that comprise nano-sized droplets which are dispersed in an outer, continuous, aqueous phase. The droplets comprise a lipophilic core, often referred to as inner phase, surrounded by amphiphilic molecules called surfactants. The droplets of inner phase and surfactants are called micelles. The micelles have an average size below about 100nm, usually determined by dynamic light scattering (DLS) technique. The micelles might therefore also be referred to as nanomicelles.

The nanoemulsions described herein comprise nanomicelles, the micelles comprising active pharmaceutical ingredients (APIs), a surfactant, a solvent, and an oil. Charged, hydrophilic moieties, often called head groups, of components in the nanomicelles are oriented towards the outer aqueous phase, whilst uncharged, lipophilic moieties are directed towards the core of the nanomicelles.

Nanoemulsions are very well suited for the formulation of tailored pharmaceutical compositions for topical drug delivery. Additional ingredients may be incorporated within the core of the nanomicelles, in an area close to the charged head groups of the complex, at an interphase between the nanomicelle and the outer phase, or in the aqueous phase. Where an ingredient will accumulate depends on the exact composition, and the characteristics of the additional ingredient, such as hydrophilic lipophilic balance (HLB), partition coefficient and polarity. However, incorporating an additional ingredient, and even changing the concentration of an ingredient, can also destabilise the system, leading to flocculation, emulsion breaking, coalescence from nano-micelles to larger droplets, creaming or the like. Therefore, nanoemulsions are a very delicate pharmaceutical form.

The compositions disclosed herein comprise a combination of diclofenac alkali metal salt (component **a)** ) and diclofenac free acid (component **b)** ) as the active pharmaceutical ingredients (APIs). Diclofenac sodium and diclofenac potassium salts are especially useful. Most preferred is diclofenac sodium.

Preferably, diclofenac and diclofenac alkali metal salt are the only API in the compositions. In one embodiment, the ratio of diclofenac alkali metal salt to diclofenac free acid is about 0.8 : 1.2 to about 1.2 : 0.8 (w:w). In another embodiment, the ratio is about 1:1 (w:w). In one embodiment, the diclofenac alkali metal salt is present in a concentration of about 0.7% to about 1.3% (w/w). In another embodiment, the diclofenac free acid is present in a concentration of about 0.7% to about 1.3% (w/w). In one embodiment, the nanoemulsion comprises diclofenac free acid in a concentration of about 1% (w/w) and diclofenac sodium salt in a concentration of about 1% (w/w). Diclofenac alkali metal salt dissociates in solution forming a diclofenac anion and an alkali metal cation. The diclofenac anion is important for the formation of the nanomicelles. The diclofenac free acid can accumulate in the core of the nanomicelle, thereby raising the overall content of diclofenac in the composition, surprisingly without hampering or destabilising the formation of the nanomicelles and the overall composition.

The nanoemulsion of this disclosure comprises a solvent (component **c)** ). The solvent is a non-volatile solvent. Preferably, the solvent is a solvent of the glycol, or glycol ether or glycol ether ester family. Preferably, the solvent has a boiling point of above about 150°C at normal pressure. Preferably, the solvent has an octanol/water partition coefficient expressed as log P_{ow} of about -0.5 to about 1 at 20°C. In one embodiment, the solvent is diethylene glycol monoethyl ether. The solvent is able to solubilise the APIs, preventing crystallisation.

In one embodiment, the solvent is present in a concentration of about 3% to about 7% (w/w). In a preferred embodiment, the solvent is present in a concentration of about 5% (w/w).

The nanoemulsion compositions can optionally comprise a cationic surfactant (component **g)** ). The cationic surfactant may have one positive charge per molecule or may have multiple positive charges per molecule. Preferably, the cationic surfactant has one positive charge per molecule.

The cationic surfactant is a cationic oil-in-water surfactant. In one embodiment, the cationic surfactant is a quaternary ammonium compound. In one embodiment, the cationic surfactant is an alkyl trimethylammonium or a dialkyl dimethylammonium salt or a combination thereof. Preferred are alkyl trimethylammonium salts. In one embodiment, the cationic surfactant is selected from the group of benzalkonium salt, benzethonium salt, cetylalkonium salt, cetylpyridinium salt, cetyltrimethylammonium salt, dequalinium salt, cetrimide and combinations thereof. In a preferred embodiment, the cationic surfactant is a C₈ to C₁₆-alkyl trimethylammonium salt. More preferred is C₁₁ to C₁₃ trimethylammonium salt. In a preferred embodiment, the cationic surfactant is cetrimide.

In one embodiment, the cationic surfactant is present in a concentration of about 0.1% to about 0.3% (w/w).

The nanoemulsion of this disclosure comprises a combination of non-ionic surfactant and a specific oil (components **d)** and **e)** ). The nonionic surfactant is involved in the formation of the nanomicelles in the nanoemulsion. In one embodiment, the nanoemulsion comprises a nonionic surfactant with a hydrophilic lipophilic balance (HLB) value of about 14-16.

In one embodiment, the nonionic surfactant is a mixture of polyethylene glycol mono- and diesters of 12-hydroxystearic acid comprising about 20% to about 40% (w/w) free polyethylene glycol. Alternatively, the nonionic surfactant can be Macrogol-15-Hydroxystearate.

In one embodiment, the nonionic surfactant is present in a concentration of about 20% to about 40% (w/w). In another embodiment, the nonionic surfactant is present in a concentration of about 25% to about 35% (w/w).

In another embodiment, the nonionic surfactant is present in a concentration of about 30% (w/w).

The oil is caprylic capric triglyceride with a caprylic acid percentage of about 55% to about 60% (w/w) and a capric acid percentage of about 40% to about 45% (w/w). The oil has a high solubilisation capacity for diclofenac. It has been found the specific ratio of fatty acids in this oil provides for stable nano-micelles, and a slight change in fatty acid composition of the used oil can destabilise the formulation, leading to phase separation. In one embodiment, the oil is Labrafac Lipophile WL1349.

In one embodiment, the oil is present in a concentration of about 2.5% to about 7.25% (w/w). In another embodiment, the oil is present in a concentration of about 4% to about 6% (w/w). In another embodiment, the oil is present in a concentration of about 5% (w/w).

The outer phase of the nanoemulsions is f), water. It may be pharma grade water or purified water or any other water suitable for pharmaceutical compositions. In one embodiment, the nanoemulsions comprises a concentration of at least about 50% (w/w) water. In another preferred embodiment, the nanoemulsions comprises a concentration of about 50% to about 60% (w/w) water. The outer phase can optionally comprise additional hydrophilic ingredients.

In one embodiment, the nanoemulsion does not contain a volatile alcohol, such as ethanol or isopropanol. C₂ to C₃ alcohols are often incorporated into topical pharmaceutical compositions to increase the solubilisation capacity of the composition for the API. However, these agents may not be preferred ingredients because of their potential for skin irritation. Furthermore, volatile alcohols may solubilise the APIs well, however they evaporate quickly upon application onto the skin, which can lead to cristallisation of the API on the skin. Preferably, the APIs are in fully solubilised state in the disclosed nanoemulsions, without the need to incorporate an alcohol.

Preferably, the nanoemulsion is translucent or transparent. In one preferred embodiment, the nanoemulsion is transparent. Translucent or transparent compositions are often preferred by patients and can lead to a better compliance. Nanoemulsions with an average micelle size, determined by dynamic light scattering, of about 10nm to about 50nm, can be regarded as translucent. Nanoemulsions with an average micelle size, determined by dynamic light scattering, of about 5nm to 10nm, can be regarded as transparent. Average micelle size is the average size of the micelles present in the nanoemulsion, determined by dynamic light scattering.

The nanoemulsions may comprise further excipients, such as perfumes, chelating agents, preservatives, antioxidants or the like.

Preferably, the nanoemulsion is self-assembling. In other words, surfactant molecules in the described composition aggregate into a micelle structure upon mixture of the ingredients. This process involves complex interfacial hydrodynamic phenomena and depends on the system composition properties. Only very specific pharmaceutical excipient combinations and concentrations lead to efficient self-assembly into nanoemulsions. Self-assembling formulations are preferred because they are more stable, less susceptible of phase separation, creaming, flocculation and the like. They are also preferred because these do not require high energy manufacturing methods like micro fluidization, high pressure homogenization, ultrasonic treatment or the like.

Preferably, the compositions have a pH suitable for topical administration. Preferably, the pH is in the range of about 8 to about 8.5. Preferably, the pH is inherent to the compositions without addition of an acid, base or buffer.

### METHODS OF MANUFACTURE

The compositions of this disclosure may be prepared as follows. Components a) to e) and optionally g) are weighed into a vessel and mixed (about 700 rotations per minute), for about 30 minutes at about 40°C to form a homogenous mixture.

Component f), water, is added under stirring at about 700 rpm for about 30 minutes at about 40°C.

### METHODS OF TREATMENT

The references to the methods of treatment by therapy of this description are to be interpreted as references to compounds, pharmaceutical compositions and medicaments of the present invention for use in those methods.

The nanoemulsions may be administered to mammals suffering from joint pain, osteoarthritis, muscle pain, back pain and/or inflammation. The nanoemulsions are useful in the treatment of joint pain, osteoarthritis, muscle pain, back pain and/or inflammation. The methods of treatment can comprise administering to a subject in need thereof a pharmaceutically effective amount of the nanoemulsion disclosed herein. The method can comprise administering the nanoemulsion to skin that covers a body part that suffers from or causes one or more of joint pain, osteoarthritis, muscle pain, back pain and/or inflammation. The method can comprise rubbing in until the skin feels dry. The method can comprise applying the nanoemulsion 2-4 times per day. Alternatively, they may be applied twice per day. Typical doses of the nanoemulsion comprise 0.09mg diclofenac equivalent/ cm² (2-4 times per day application) or 0.21mg diclofenac equivalent/ cm² (twice per day application).

The nanoemulsions may be provided in a container comprising an applicator for direct application onto the skin. The nanoemulsions may be provided in a package comprising a means for dosing. In one embodiment, the dosing means is a pump. In one embodiment, the means for dosing is a pipette.

### EXAMPLE

**Table 1: Formulation of exemplary nanoemulsion**

| Ingredient | Concentration [%w/w] |
|---|---|
| Diclofenac sodium | 1.074 |
| Diclofenac free acid | 1 |
| Labrafac WL1349 | 5 |
| Kolliphor HS15 | 30 |
| Transcutol P | 5 |
| Cetrimide | 0.25 |
| Ultrapure water | q.s. up to 100 |

The formulation was prepared as follows:
The diclofenac sodium diclofenac free acid, Labrafac^{™} WL1349, Transcutol^{®} P, Kolliphor^{®} HS15 and Cetrimide were weighed into a glass vial. The vial was heated to 40°C under continuous stirring, with a 10nm magnetic stirring bar, in a magnetic heat stirrer, during 30 minutes at 700 rpm until a homogenous solution is formed. The water was weighed into a separate vial and added at once under stirring with 700 rpm at 40°C. Stirring was continued for 30 minutes at the same temperature. The vial was closed with a cap during the stirring.

All ranges disclosed herein are to be understood to encompass any and all subranges subsumed therein. For example, a stated range of "1.0 to 10.0" should be considered to include any and all subranges beginning with a minimum value of 1.0 or more and ending with a maximum value of 10.0 or less, e.g., 1.0 to 5.3, or 4.7 to 10.0, or 3.6 to 7.9.

All ranges disclosed herein are also to be considered to include the end points of the range, unless expressly stated otherwise. For example, a range of "between 5 and 10" or "5 to 10" or "5-10" should generally be considered to include the end points 5 and 10.

## Claims

1. A nanoemulsion for topical drug delivery, comprising:
**a)** diclofenac alkali metal salt, in a concentration of 0.7% to 1.3% (w/w),
**b)** diclofenac free acid, in a concentration of 0.7% to 1.3% (w/w),
**c)** diethyleneglycol monoethyl ether, in a concentration of 3% to 7% (w/w),
**d)** a mixture of polyethylene glycol mono- and diesters of 12-hydroxystearic acid comprising 20% to 40% (w/w) free polyethylene glycol,
**e)** caprylic capric triglyceride with a caprylic acid percentage of 55% to 60% (w/w), and a capric acid percentage of 40% to 45% (w/w), and
**f)** water.

2. A nanoemulsion according to claim 1, wherein the ratio of diclofenac alkali metal salt to diclofenac free acid is 0.8 : 1.2 to 1.2 : 0.8 (w:w).

3. A nanoemulsion according to claim 1 or 2, wherein the total concentration of diclofenac is from 1.5% to 2.5% (w/w).

4. A nanoemulsion according to any of claims 1, 2 or 3, further comprising
**g)** a cationic surfactant, in a concentration of 0.1% to 0.3% (w/w).

5. A nanoemulsion according to any of the preceding claims, which is self-emulsifying.

6. A nanoemulsion according to any of the preceding claims, which is transparent.

7. A nanoemulsion according to any of the preceding claims, wherein the nanoemulsion is alcohol-free.

8. A nanoemulsion according to any of the preceding claims, wherein component g) is a quaternary ammonium compound.

9. A nanoemulsion according to claim 8, wherein component g) is a C₈-C₁₆-alkyl trimethylammonium salt.

10. A nanoemulsion according to any of the preceding claims, wherein component d) is present in a concentration of 20% to 40% (w/w) and component e) is present in a concentration of 2.5% to 7.25% (w/w).

11. A nanoemulsion according to any of the preceding claims, for use in the treatment of joint pain, osteoarthritis, muscle pain, back pain and/or inflammation.

12. A composition according to any of claims 1 to 10, provided in a container comprising an applicator for direct application onto the skin.

## Patentansprüche

1. Nanoemulsion zur topischen Wirkstoffverabreichung, umfassend:
a) Diclofenac-Alkalimetallsalz, in einer Konzentration von 0,7% bis 1,3% (w/w),
b) freie Säure von Diclofenac, in einer Konzentration von 0,7% bis 1,3% (w/w),
c) Diethylenglycolmonoethylether, in einer Konzentration von 3% bis 7% (w/w),
d) eine Mischung aus Polyethylenglykolmono- und - diestern der 12-Hydroxystearinsäure, umfassend 20% bis 40% (w/w) freies Polyethylenglykol,
e) Capryl-Capric-Triglycerid mit einem Caprylsäureanteil von 55% bis 60% (w/w) und einem Caprinsäureanteil von 40% bis 45% (w/w), und
f) Wasser.

2. Nanoemulsion gemäß Anspruch 1, worin das Verhältnis von Diclofenac-Alkalimetallsalz zur freien Säure von Diclofenac 0,8 : 1,2 bis 1,2 : 0,8 (w/w) beträgt.

3. Nanoemulsion gemäß Anspruch 1 oder 2, worin die Gesamtkonzentration von Diclofenac 1,5% bis 2,5% (w/w) beträgt.

4. Nanoemulsion gemäß einem der Ansprüche 1, 2 oder 3, ferner umfassend g) ein kationisches Tensid, in einer Konzentration von 0,1% bis 0,3% (w/w).

5. Nanoemulsion gemäß einem der vorangehenden Ansprüche, die selbstemulgierend ist.

6. Nanoemulsion gemäß einem der vorangehenden Ansprüche, die transparent ist.

7. Nanoemulsion gemäß einem der vorangehenden Ansprüche, worin die Nanoemulsion alkoholfrei ist.

8. Nanoemulsion gemäß einem der vorangehenden Ansprüche, worin die Komponente g) eine quaternäre Ammoniumverbindung ist.

9. Nanoemulsion gemäß Anspruch 8, worin die Komponente g) ein C₈-C₁₆-Alkyltrimethylammoniumsalz ist.

10. Nanoemulsion gemäß einem der vorangehenden Ansprüche, worin die Komponente d) in einer Konzentration von 20% bis 40% (w/w) vorliegt und die Komponente e) in einer Konzentration von 2,5% bis 7,25% (w/w) vorliegt.

11. Nanoemulsion gemäß einem der vorangehenden Ansprüche zur Verwendung bei der Behandlung von Gelenkschmerzen, Osteoarthritis, Muskelschmerzen, Rückenschmerzen und/oder Entzündungen.

12. Zusammensetzung gemäß einem der Ansprüche 1 bis 10, bereitgestellt in einem Behälter, umfassend einen Applikator zur direkten Anwendung auf die Haut.

## Revendications

1. Nanoémulsion pour l'administration topique de médicament, comprenant:
a) un sel de métal alcalin et de diclofénac, à une concentration de 0,7 % à 1,3 % (m/m),
b) du diclofénac acide libre, à une concentration de 0,7 % à 1,3 % (m/m),
c) de l'éther monoéthylique de diéthylèneglycol, à une concentration de 3 % à 7 % (m/m),
d) un mélange de mono- et diesters de polyéthylène glycol et d'acide 12-hydroxystéarique comprenant 20 % à 40 % (m/m) de polyéthylène glycol libre,
e) un triglycéride caprylique-caprique avec un pourcentage de 55 % à 60 % d'acide caprylique et un pourcentage de 40 % à 45 % (m/m) d'acide caprique, et
f) de l'eau.

2. Nanoémulsion selon la revendication 1, dans laquelle le rapport du sel de métal alcalin et de diclofénac au diclofénac acide libre est 0,8 : 1,2 à 1,2 : 0,8 (m/m).

3. Nanoémulsion selon la revendication 1 ou 2, dans laquelle la concentration totale de diclofénac est de 1,5 % à 2,5 % (m/m).

4. Nanoémulsion selon l'une quelconque des revendications 1, 2 ou 3, comprenant en outre
g) un tensioactif cationique, à une concentration de 0,1 % à 0,3 % (m/m).

5. Nanoémulsion selon l'une quelconque des revendications précédentes, qui est auto-émulsifiante.

6. Nanoémulsion selon l'une quelconque des revendications précédentes, qui est transparente.

7. Nanoémulsion selon l'une quelconque des revendications précédentes, dans laquelle la nanoémulsion est sans alcool.

8. Nanoémulsion selon l'une quelconque des revendications précédentes, dans laquelle le composant g) est un composé d'ammonium quaternaire.

9. Nanoémulsion selon la revendication 8, dans laquelle le composant g) est un sel de C₈-C₁₆ alkyl triméthylammonium.

10. Nanoémulsion selon l'une quelconque des revendications précédentes, dans laquelle le composant d) est présent à une concentration de 20 % à 40 % (m/m) et le composant e) est présent à une concentration de 2,5 % à 7,25 % (m/m).

11. Nanoémulsion selon l'une quelconque des revendications précédentes, destinée à être utilisée dans le traitement des douleurs articulaires, de l'arthrose, des douleurs musculaires, des lombalgies et/ou des inflammations.

12. Composition selon l'une quelconque des revendications 1 à 10, fournie dans un récipient comprenant un applicateur pour l'application directe sur la peau.
